# EUROPÄISCHE PATENTANMELDUNG

(11) **EP 1 743 577 A1**
(43) Veröffentlichungstag der Anmeldung: **17.01.2007**
(21) Anmeldenummer: 05013559.9
(22) Anmeldetag: 23.06.2005
(51) Int. Cl.: A61B 5/151, A61B 5/157

(54) **Analysehandgerät zum Untersuchen von Körperflüssigkeiten**

(71) Anmelder: Roche Diagnostics GmbH, 68305 Mannheim (DE); F. HOFFMANN-LA ROCHE AG, 4070 Basel (CH)
(72) Erfinder: Kistner, Michael, 67017 Ludwigshafen (DE); Deck, Frank, 67150 Niederkirchen (DE)
(74) Vertreter: Pfeifer, Hans-Peter

(57) **Zusammenfassung**

Die Erfindung betriff ein Analysehandgerät zum Untersuchen von Körperflüssigkeit, umfassend ein Gehäuse (4) mit einer Gehäuseöffnung (5), an die ein Körperteil, insbesondere ein Finger, zum Erzeugen einer Einstichwunde anlegbar ist, eine Analyseeinrichtung (2) zum Untersuchen einer an der Einstichwunde gewonnenen Probe einer Körperflüssigkeit, eine Stecheinheit (3), die eine Lanzette und einen Lanzettenantrieb zum Erzeugen einer Einstich- und Rückführbewegung der Lanzette umfaßt, eine Transporteinrichtung, mit der die Stecheinheit (3) zwischen einer Arbeitsposition, in der mit der Lanzette eine Einstichwunde in einem an der Gehäuseöffnung (5) anliegenden Körperteil erzeugbar ist, und einer Ruheposition beweglich ist, und eine Bedienungseinrichtung (10a,10b,10c,10d) zum Betätigen eines Funktionsmechanismus (11a,11b,11c,11d) der Stecheinheit (3). Erfindungsgemäß ist vorgesehen, daß die Stecheinheit (3) in einer ihrer beiden Positionen von der Bedienungseinrichtung (10a,10b,10c,10d) entkoppelt ist und in der anderen Position mit der Bedienungseinrichtung (10a,10b,10c,10d) in Wirkeingriff steht.

## Beschreibung

Die Erfindung betrifft ein Analysehandgerät zum Untersuchen von Körperflüssigkeit, umfassend ein Gehäuse mit einer Gehäuseöffnung, an die ein Körperteil, insbesondere ein Finger, zum Erzeugen einer Einstichwunde anlegbar ist, eine Analyseeinrichtung zum Untersuchen einer Körperflüssigkeit, die nach der Erzeugung einer Einstichwunde aus dieser gewonnen wurde, eine Stecheinheit, die eine Lanzette und einen Lanzettenantrieb zum Erzeugen einer Einstich- und Rückführbewegung der Lanzette umfaßt, eine Transporteinrichtung, mit der die Stecheinheit zwischen einer Arbeitsposition, in der mit der Lanzette eine Einstichwunde in einem an der Gehäuseöffnung anliegenden Körperteil erzeugbar ist, und einer Ruheposition beweglich ist, und eine Bedienungseinrichtung zum Betätigen eines Funktionsmechanismus der Stecheinheit. Ein derartiges Analysehandgerät ist aus der DE 10332488 A1 bekannt.

Je nach Tiefe der Einstichwunde handelt es sich bei der Körperflüssigkeit um interstitielle Flüssigkeit und/oder Blut. Nachfolgend wird ohne Beschränkung der Allgemeinheit auf Blut als Beispiel der zu untersuchenden Körperflüssigkeit Bezug genommen.

Mit Analysehandgeräten, die zusätzlich zu einer Analyseeinrichtung zum Untersuchen von Blut eine Stecheinheit zum Erzeugen einer Einstichwunde enthalten, läßt sich eine Messung des Blutzuckerspiegels wesentlich einfacher durchführen als mit Analysesystemen, die aus einem Analysehandgerät, einem separaten Lanzettengerät und Teststreifen für die Blutuntersuchung bestehen.

Bei Analysesystemen mit separaten Geräten muß ein Benutzer zuerst mit einem Lanzettengerät eine Einstichwunde erzeugen, anschließend austretendes Blut auf einen Teststreifen aufbringen und diesen schließlich für die eigentliche Messung der Blutglucosekonzentration in ein Analysehandgerät einführen.

Mit integrierten Analysehandgeräten der eingangs genannten Art wird der Vorgang der Blutgewinnung und Messung für den Benutzer wesentlich vereinfacht. Es genügt, das Analysehandgerät mit seiner Gehäuseöffnung an einen Finger anzulegen. Mit der in das Gerät integrierten Stecheinheit wird eine Einstichwunde erzeugt. Anschließend austretendes Blut gelangt ohne weiteres Zutun des Benutzers zu der Analyseeinrichtung und wird von dieser untersucht. Dabei befindet sich die Stecheinheit um Erzeugen der Einstichwunde zunächst in ihrer Arbeitsposition an der Gehäuseöffnung. Anschließend wird sie von einer Transporteinrichtung in eine Ruheposition bewegt, so daß die Gehäuseöffnung und damit die erzeugte Einstichwunde für eine Probenaufnahme zugänglich sind.

Da Diabetiker ihren Blutzuckerspiegel mehrmals täglich messen und deshalb entsprechende Analysehandgeräte ständig mit sich führen müssen, besteht die Forderung, Analysehandgeräte möglichst klein und kompakt zu fertigen. Wichtig in diesem Zusammenhang ist ein möglichst geringer Energieverbrauch, da die Leistung von internen Stromquellen begrenzt ist. Häufige Batteriewechsel schränken den Benutzerkomfort ein. Größere Batterien sind mit der Forderung einer kompakten Bauweise des Analysehandgerätes nicht vereinbar.

Aufgabe der Erfindung ist es deshalb, einen Weg aufzuzeigen, wie ein Analysehandgerät der eingangs genannten Art kompakter gefertigt und ohne Einschränkung des Benutzerkomforts mit geringerem Energieaufwand betrieben werden kann.

Diese Aufgabe wird erfindungsgemäß dadurch gelöst, daß die Stecheinheit in einer ihrer beiden Positionen von der Bedienungseinrichtung entkoppelt ist und in der anderen Position mit der Bedienungseinrichtung gekoppelt ist, vorzugsweise mit ihr mechanisch in Wirkeingriff steht.

Die Stecheinheit eines Analysehandgerätes hat typischerweise mehrere Funktionsmechanismen, die beispielsweise dem Einstellen der Stechtiefe oder dem Spannen einer Antriebsfeder dienen. Für jeden dieser Funktionsmechanismen ist eine Bedienungseinrichtung erforderlich, damit der entsprechende Funktionsmechanismus betätigt werden kann. Die Bedienungseinrichtung weist ein entsprechendes Bedienungselement, beispielsweise einen Drehknopf oder eine Taste, für den Benutzer auf.

Bevorzugt ist die Stecheinheit in ihrer Ruheposition mit der Betätigungseinrichtung, beispielsweise zum Spannen einer Antriebsfeder oder dem Einstellen der Stechtiefe, gekoppelt und in ihrer Arbeitsposition von der Betätigungseinrichtung entkoppelt. Möglich ist es aber auch, daß die Stecheinheit in ihrer Ruheposition von der Bedienungseinrichtung entkoppelt ist und in der Arbeitsposition mit der Bedienungseinrichtung in Wirkeingriff steht.

Mit der Erfindung läßt sich der Energiebedarf des Analysehandgerätes senken, da nur die Stecheinheit selbst und nicht zusätzlich auch noch die Bedienungseinrichtung zwischen der Ruheposition und der Arbeitsposition hin und her bewegt werden muß. Die zu bewegende Masse wird auf ein Minimum reduziert, so daß der Leistungsbedarf der Transporteinheit entsprechend gering ist. Als weiterer Vorteil kommt hinzu, daß bei einem erfindungsgemäßen Gerät die Gefahr von Fehlbedienungen reduziert ist, da der Funktionsmechanismus nur dann betätigt werden kann, wenn er mit der Bedienungseinrichtung gekoppelt ist. Ist der Funktionsmechanismus von der Bedienungseinrichtung entkoppelt, so hat eine fehlerhafte Betätigung der Bedienungseinrichtung keine Auswirkungen auf den Funktionsmechanismus.

Die erfindungsgemäße Entkopplung der Bedienungseinrichtung von der Stecheinheit ermöglicht ferner eine kompaktere Bauweise des Analysehandgerätes. Dies liegt daran, daß in dem Gehäuse ein relativ kleiner Freiraum ausreicht, in dem die Stecheinheit zwischen der Arbeitsposition und der Ruheposition bewegt werden kann und kein zusätzlicher Freiraum für eine entsprechende Bewegung der Bedienungseinrichtung erforderlich ist.

Bei der bisher gebräuchlichen permanenten Kopplung der Bedienungseinrichtung mit der Stecheinheit müssen im Falle mechanischer Bedienungselemente, beispielsweise mittels Drehknöpfen bewegliche Wellen, durch Schlitze in der Gehäusewand hindurch geführt werden, damit sie zusammen mit der Stecheinheit beweglich sind. Derartige Schlitze haben den Nachteil, daß durch sie Schmutz in das Gehäuseinnere gelangen kann. Anstatt an dem bewerten Konzept einer permanenten Kopplung festzuhalten und nachteilige Schlitze beispielsweise durch elektrische Betätigungselemente, die über flexible Drähte mit beweglichen Teilen der Bedienungseinrichtung verbunden sind, zu vermeiden, geht die Erfindung mit einer Bedienungseinrichtung, die je nach Position der Stecheinheit mit ihr gekoppelt oder entkoppelt ist, einen neuen Weg. Auf diese Weise können mit einer relativ einfachen mechanischen Konstruktion die oben genannten Vorteile einer kompakteren Bauweise und einem geringen Stromverbrauch genutzt werden.

Die Aussage, daß die Stecheinheit von der Bedienungseinrichtung entkoppelt ist, ist im Rahmen der vorliegenden Anmeldung so zu verstehen, daß in diesem Zustand keine Kraftübertragung von der Bedienungseinrichtung auf den Funktionsmechanismus der Stecheinheit möglich ist. Bevorzugt ist in dem entkoppelten Zustand auch kein Kontakt zwischen der Bedienungseinrichtung und der Stecheinheit vorhanden. Eine Kopplung kann im einfachsten Fall durch das Schließen eines elektrischen Kontakts erfolgen, geschieht jedoch bevorzugt dadurch, daß die Bedienungseinrichtung mit dem Funktionsmechanismus mechanisch in Wirkeingriff tritt. Steht die Bedienungseinrichtung mit der Stecheinheit in Wirkeingriff, so kann mechanisch eine Kraft von der Bedienungseinrichtung auf den Funktionsmechanismus der Stecheinheit übertragen werden. Bevorzugt wird zum Betätigen des Funktionsmechanismus über den Wirkeingriff eine Drehbewegung, beispielsweise über Wellen oder Zahnräder, von der Bedienungseinrichtung auf den Funktionsmechanismus übertragen. Dies ist durch eine kraftschlüssige oder - bevorzugt - durch eine formschlüssige Kopplung möglich.

Weitere Einzelheiten und Vorteile der Erfindung werden im folgenden anhand eines Ausführungsbeispiels unter Bezugnahme auf die beigefügte Figuren näher erläutert. Die darin dargestellten Besonderheiten können einzeln oder in Kombination verwendet werden, um bevorzugte Ausgestaltungen zu schaffen. Es zeigen:
- Fig. 1: eine schematische Darstellung eines Analysehandgerätes bei aufgeschnittenem Gehäuse;
- Fig. 2: eine Detailansicht des in Figur 1 gezeigten Analysehandgerätes, in der sich die Stecheinheit in der Arbeitsposition befindet; und
- Fig. 3: eine Detailansicht der Stecheinheit in der Ruheposition.

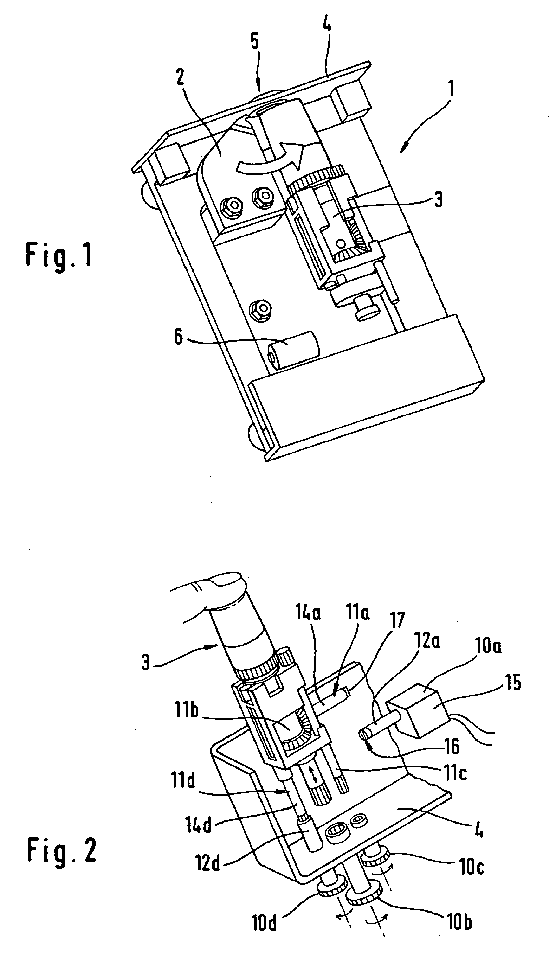

In Figur 1 ist schematisch ein Ausführungsbeispiel eines Analysehandgerätes 1 dargestellt, das eine Analyseeinrichtung 2 zur Untersuchung von Blut und eine Stecheinheit 3 zum Erzeugen einer Einstichwunde umfaßt. Die Stecheinheit 3 umfaßt eine Lanzette (nicht dargestellt) und einen Lanzettenantrieb (nicht dargestellt) zum Erzeugen einer Einstich- und Rückführbewegung der Lanzette. Das Analysehandgerät 1 hat ein,Gehäuse 4 mit einer Gehäuseöffnung 5, an die ein Körperteil, beispielsweise ein Finger zum Erzeugen einer Einstichwunde angelegt wird.

Befindet sich die Stecheinheit 3 in der in Figur 1 dargestellten Arbeitsposition, so kann eine Einstichwunde in einem an der Gehäuseöffnung 5 anliegenden Körperteil erzeugt werden. Nach einem Einstich wird die Stecheinheit 3 mittels einer Transporteinrichtung (nicht dargestellt) durch eine Schub- oder Schwenkbewegung in eine Ruheposition befördert. Die Analyseeinrichtung 2 wird in den dabei freiwerdenden Raum zu der Gehäuseöffnung 5 bewegt, so daß die Analyseeinrichtung 2 in eine Position für die Aufnahme von Blut gelangt, das aus der erzeugten Einstichwunde ausgetreten ist.

Bei dem dargestellten Ausführungsbeispiel enthält die Analyseeinrichtung 2 eine Kassette mit einem als Band ausgeführten Teststreifen. Der Teststreifen weist eine Vielzahl von Bandabschnitten auf, die mit Chemikalien beschichtet sind, die mit aufgebrachtem Blut reagieren und dabei eine der Blutzuckerkonzentration entsprechende, optisch detektierbare Farbänderung bewirken. Die Analyseeinrichtung 2 und sonstige elektrischen Baueinheiten des Analysehandgeräts 1 werden von einer internen Stromquelle 6 in Form einer handelsüblichen Batterie mit Energie versorgt.

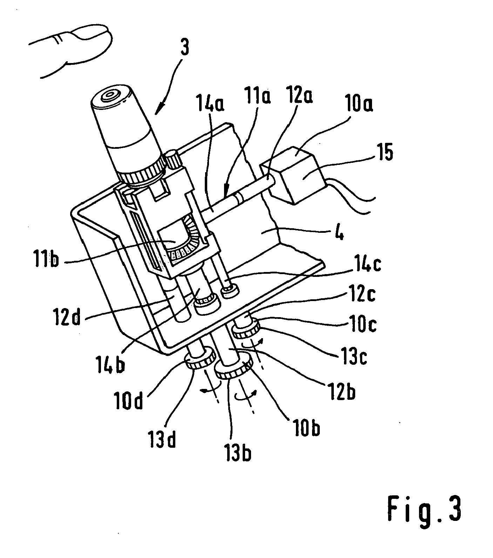

In Figur 1 sind der besseren Übersichtlichkeit wegen keine Bedienungseinrichtungen zum Betätigen von Funktionsmechanismen der Stecheinheit 3 dargestellt. Diese Bedienungseinrichtungen und Funktionsmechanismen der Stecheinheit 3 werden im Folgenden anhand der Figuren 2 und 3 erläutert.

In Figur 2 ist die Stecheinheit 3 in der Arbeitsposition zusammen mit verschiedenen Bedienungseinrichtungen 10a,10b,10c,10d dargestellt, die zum Betätigen von Funktionsmechanismen 11a,11b,11c,11d der Stecheinheit 3 dienen. Bei dem dargestellten Ausführungsbeispiel hat die Stecheinheit 3 einen ersten Funktionsmechanismus 11a zum Spannen einer Antriebsfeder, einen zweiten Funktionsmechanismus 11c zum Einstellen der Stechtiefe, einen dritten Funktionsmechanismus 11b zum Drehen eines Lanzettenmagazins, das in der Stecheinheit 3 gelagert ist, und einen vierten Funktionsmechanismus 11d zum Auslösen einer Einstich- und Rückführbewegung einer Lanzette.

Eine Stecheinheit mit derartigen Funktionsmechanismen ist im Stand der Technik bekannt und beispielsweise in der deutschen Patentanmeldung 102004059491.0 beschrieben, die diesbezüglich durch Bezugnahme zum Gegenstand der Anmeldung gemacht wird.

In der in Figur 2 dargestellten Arbeitsposition der Stecheinheit 3 steht nur die Bedienungseinrichtung 10d mit der Stecheinheit 3, genauer gesagt dem Funktionsmechanismus 11d, in Wirkeingriff. Die übrigen Bedienungseinrichtungen 10a,10b,10c sind von der Stecheinheit 3, genauer gesagt den dazugehörenden Funktionsmechanismen 11a,11b bzw. 11c, entkoppelt. In Figur 3 ist die Stecheinheit 3 in ihrer Ruheposition dargestellt. In der Ruheposition stehen die Bedienungseinrichtungen 10a,10b und 10c mit der Stecheinheit 3 in Wirkeingriff, während die Bedienungseinrichtung 10d von der Stecheinheit 3 entkoppelt ist.

Der Wirkeingriff der Stecheinheit 3 mit den Bedienungseinrichtungen 10a,10b,10c,10d dient dem Übertragen von Drehbewegungen von der jeweiligen Bedienungseinrichtung 10a,10b,10c,10d auf den dazugehörenden Funktionsmechanismus 11a,11b,11c,11d der Stecheinheit 3. Jede Bedienungseinrichtung 10a,10b,10c,10d umfaßt zu diesem Zweck eine Welle 12a,12b,12c bzw. 12d, die formschlüssig mit dem jeweiligen Funktionsmechanismus 11a,11b,11c bzw. 11d der Stecheinheit 3 gekoppelt werden kann.

Zu der Bedienungseinrichtung 10d gehört eine durch die Außenwand des Gehäuses 4 hindurchragende Welle 12d, die an ihren aus dem Gehäuse 4 herausragenden Ende einen Drehknopf 13d trägt. Die Welle 12d ist als Hohlwelle ausgeführt, so daß sie auf eine passende Welle 14d (Figur 2) des Funktionsmechanismus 11d, mit dem die Stecheinheit 3 ausgelöst werden kann, aufgeschoben werden kann. Die beiden Wellen 12d und 14d sind in der in Figur 2 dargestellten Arbeitsposition der Stecheinheit 3 über eine Keilwellenverbindung verbunden. Dabei sind die Keilnaben nur in einem vorderen Abschnitt der Welle 12d vorhanden. Der Innendurchmesser der Hohlwelle 12d nimmt hinter diesem Abschnitt mit den Keilnaben etwas zu, so daß in der in Figur 3 dargestellten Ruheposition der Stecheinheit 3 keine Drehbewegung von der Welle 12d auf die Welle 14d übertragen werden kann. Die Bedienungseinrichtung 10d also von dem Funktionsmechanismus 11d entkoppelt ist.

Der Aufbau der Bedienungseinrichtungen 10b und 10c entspricht dem Aufbau der Bedienungseinrichtung 10d. Die Bedienungseinrichtungen 10b und 10c umfassen jeweils eine durch eine Außenwand des Gehäuses 4 hindurchragende Welle 12b bzw. 12c, an deren Ende ein für ein Benutzer zugänglicher Drehknopf 13b bzw. 13c angebracht ist. Die Wellen 12b und 12c treten mit entsprechenden Wellen 14b bzw. 14c der Funktionsmechanismen 11b bzw. 11c über eine Keilwellenverbindung in Wirkeingriff, sobald sich die Stecheinheit 3 in der in Figur 3 dargestellten Ruheposition befindet. Anstelle einer Keilwellenverbindung können zur Kopplung der Wellen 12b und 14b bzw. 12c und 14c auch andere formschlüssige Verbindungen, beispielsweise Stirnverzahnungen oder Polygonprofile verwendet werden.

Die Bedienungseinrichtung 10a, mit welcher der Funktionsmechanismus 11a zum Spannen einer Antriebsfeder der Stecheinheit 3 betätigt wird, umfaßt einen Elektromotor 15, von dem eine Welle 12a angetrieben wird, die in der in Figur 3 dargestellten Ruheposition der Stecheinheit 3 mit der Welle 14a des Funktionsmechanismus 11a gekoppelt ist. Die Bedienungseinrichtung 10a umfaßt ferner ein Bedienungselement (nicht dargestellt, beispielsweise in Form einer Taste, mit der ein Benutzer den Elektromotor einschalten kann.

Im Unterschied zu den Wellen 12b,12c und 12d verläuft die Welle 12a quer zu der Richtung, in der die Stecheinheit 3 bei ihrer Bewegung von der Ruheposition in die Arbeitsposition verschoben wird. Die Welle 12a weist deshalb an ihrem von dem Motor 15 abgewandten Ende einen Schlitz 16 auf, in den ein Steg 17 an dem freien Ende der Welle 14a nach dem Prinzip einer Nut- und Federverbindung eingreift, wenn sich die Stecheinheit 3 in der in Figur 3 dargestellten Ruheposition befindet.

### Bezugszeichenliste

- 1: Analysehandgerät
- 2: Analyseeinrichtung
- 3: Stecheinheit
- 4: Gehäuse
- 5: Gehäuseöffnung
- 6: interne Stromquelle
- 10a,10b,10c,10d: Bedienungseinrichtungen
- 11a,11b,11c,11d: Funktionsmechanismen
- 12a,12b,12c,12d: Wellen
- 13b,13c,13d: Drehknopf
- 14a,14b,14c,14d: Welle
- 15: Elektromotor
- 16: Schlitz
- 17: Steg

## Patentansprüche

1. Analysehandgerät zum Untersuchen von Körperflüssigkeit, umfassend:
ein Gehäuse (4) mit einer Gehäuseöffnung (5), an die ein Körperteil, insbesondere ein Finger, zum Erzeugen einer Einstichwunde anlegbar ist,
eine Analyseeinrichtung (2) zum Untersuchen einer an der Einstichwunde gewonnenen Probe einer Körperflüssigkeit,
eine Stecheinheit (3), die eine Lanzette und einen Lanzettenantrieb zum Erzeugen einer Einstich- und Rückführbewegung der Lanzette umfaßt,
eine Transporteinrichtung, mit der die Stecheinheit (3) zwischen einer Arbeitsposition, in der mit der Lanzette eine Einstichwunde in einem an der Gehäuseöffnung (5) anliegenden Körperteil erzeugbar ist, und
einer Ruheposition beweglich ist, und
eine Bedienungseinrichtung (10a,10b,10c,10d) zum Betätigen eines Funktionsmechanismus (11a,11b,11c,11d) der Stecheinheit (3),
**dadurch gekennzeichnet, daß** die Stecheinheit (3) in einer ihrer beiden Positionen von der Bedienungseinrichtung (10a,10b,10c,10d) entkoppelt ist und in der anderen Position mit der Bedienungseinrichtung (10a,10b,10c,10d) gekoppelt ist, vorzugsweise mechanisch mit ihr in Wirkeingriff steht.

2. Analysehandgerät nach Anspruch 1, **dadurch gekennzeichnet, daß** die Stecheinheit (3) in der Arbeitsposition von der Bedienungseinrichtung (10a,10b,10c) entkoppelt ist und in der Ruheposition mit der Bedienungseinrichtung (10a,10b,10c) in Wirkeingriff steht.

3. Analysehandgerät nach einem der vorhergehenden Ansprüche, **dadurch gekennzeichnet, daß** der Wirkeingriff der Stecheinheit (3) mit der Bedienungseinrichtung (10a,10b,10c,10d) dem Übertragen einer Drehbewegung von der Bedienungseinrichtung (10a,10b,10c,10d) auf den Funktionsmechanismus (11a,11b,11c,11d) der Stecheinheit (3) dient.

4. Analysehandgerät nach Anspruch 3, **dadurch gekennzeichnet, daß** die Bedienungseinrichtung (10a,10b,10c,10d) eine Welle (12a,12b,12c,12d) umfaßt.

5. Analysehandgerät nach Anspruch 4, **dadurch gekennzeichnet, daß** die Welle (12b,12c,12d) durch eine Außenwand des Gehäuses (4) hindurchragt.

6. Analysehandgerät nach einem der vorhergehenden Ansprüche, **dadurch gekennzeichnet, daß** der Wirkeingriff der Stecheinheit (3) mit der Bedienungseinrichtung (10a,10b,10c,10d) formschlüssig ist.

7. Analysehandgerät nach einem der vorhergehenden Ansprüche, **dadurch gekennzeichnet, daß** die Stecheinheit (3) einen durch eine Antriebsfeder antreibbaren Antriebsrotor umfaßt.

8. Analysehandgerät nach Anspruch 7, **dadurch gekennzeichnet, daß** der durch die Bedienungseinrichtung (10a) betätigbare Funktionsmechanismus (11a) der Stecheinheit (3) zum Spannen der Antriebsfeder dient.

9. Analysehandgerät nach Anspruch 8, **dadurch gekennzeichnet, daß** die Bedienungseinrichtung (10a) einen in dem Gehäuse (4) angeordneten Motor (15) zum Spannen der Antriebsfeder umfaßt.

10. Analysehandgerät nach einem der vorhergehenden Ansprüche, **dadurch gekennzeichnet, daß** der durch die Bedienungseinrichtung (10c) betätigbare Funktionsmechanismus (11c) der Stecheinheit (3) zum Einstellen der Stechtiefe dient.

11. Analysehandgerät nach einem der vorhergehenden Ansprüche, **dadurch gekennzeichnet, daß** die Stecheinheit (3) eine Aufnahme für ein Lanzettenmagazin mit mehreren Lanzetten umfaßt, die durch Drehen des Lanzettenmagazins nacheinander in eine Kopplungsposition beweglich sind, in der sie mit dem Lanzettenantrieb koppelbar sind, wobei der durch die Bedienungseinrichtung (10b) betätigbare,Funktionsmechanismus (11b) der Stecheinheit (3) zum Drehen des Lanzettenmagazins dient.

12. Analysehandgerät nach einem der vorhergehenden Ansprüche, **dadurch gekennzeichnet, daß** es mehrere Bedienungseinrichtungen (10a,10b,10c,10d) aufweist, mit denen die Stecheinheit (3) in einer ihrer beiden Positionen in Wirkeingriff steht und von denen die Stecheinheit (3) in der anderen Position entkoppelt ist.
